# EUROPEAN PATENT APPLICATION

(11) **EP 0 646 358 A1**
(43) Date of publication of application: **05.04.1995**
(21) Application number: 94114462.8
(22) Date of filing: 14.09.1994
(51) Int. Cl.: A61B 17/34, A61B 17/32

(54) **Instrument for laparoscopy**

(30) Priority: 05.10.1993 SE 9303253
(71) Applicant: Pacesetter AB, S-171 95 Solna (SE)
(72) Inventor: Bowald, Staffan, S-740 10 Almunge (SE); Hirschberg, Jakub, S-183 44 Täby (SE)
(74) Representative: Lettström, Richard Wilhelm

(57) **Abstract**

An instrument for laparoscopy comprises an introducer tool (1) devised for introduction and positional fixation of surgical equipment in the form of at least two surgical tools, optical instruments, implants or the like.

## Description

This invention relates to an instrument for laparoscopy. Laparoscopy greatly reduces surgical trauma in a number of different surgical operations.

For access to e.g. the heart for e.g. implantation of stimulation electrodes or other treatments in, on and around the heart, the heart must be exposed by thoracotomy. This constitutes major surgery for a patient with heart disease and is associated with high morbidity and mortality.

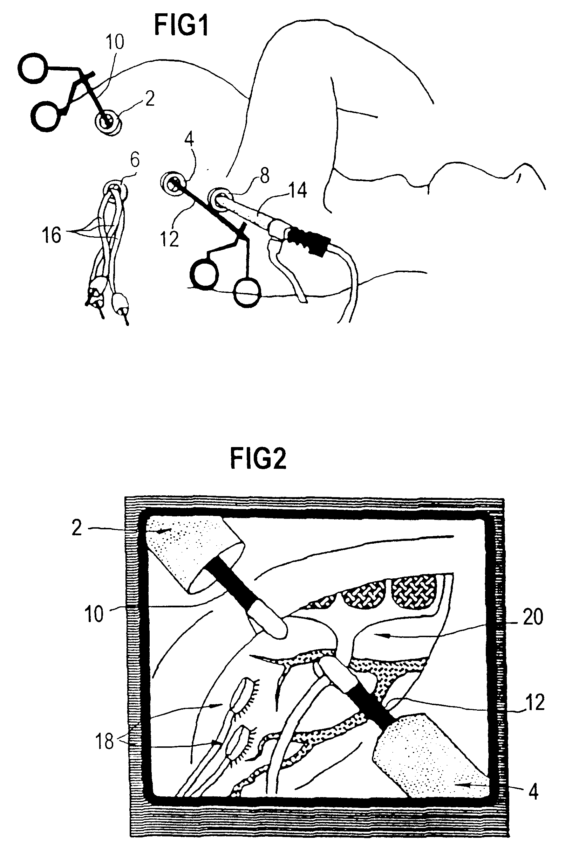

Laparoscopic technics, i.e. thoracoscopic methods, have come into use in recent years for minor thorax surgery. One example of such prior art laparoscopy in the implantation of defibrillation electrodes is illustrated in FIGS. 1 and 2.

A number of introducer tools 2, 4, 6, 8, i.e. trocars, are advanced to the pericardium through channels established in the patient. Surgical instruments, such as different gripping tools 10, 12, optical instruments 14 and implants in the form of electrodes 18, 20 with attendant leads, are then introduced through these trocars 2, 4, 6 8.

The optical instrument 14, a thoracoscope, is advanced through the pericardium, and the thoracoscope generates an image on a TV monitor (see FIG. 2) so the surface of the heart can be inspected.

The electrodes 18 are pacing electrodes, whereas the electrode 20 is a so-called patch electrode.

The object of the present invention is to further simplify present-day laparoscopy and make it even less traumatic to patients.

This object is achieved with an instrument according to claim 1.

With the instrument according to the invention, a plurality of instruments and possibly even an implant can be introduced through one and the same introducer tool which only requires a single channel for introduction into the patient. The result is shorter hospitalization and care times and a reduced risk of complications, such as puncture of the pleura and damage to the phrenic nerve during thorax surgery. The instrument also provides better access for inspection and manipulation, and implantation of e.g. cardiac stimulation electrodes is as simple as in transvenous implantation.

According to one advantageous embodiment of the instrument according to the invention, the introducer tool is devised with a central channel for the introduction of an optical instrument and additional, lateral channels for the introduction of other surgical equipment. This thereby offers the surgeon a good view for inspecting and monitoring the area of interest of the organ in question.

According to another advantageous embodiment of the instrument according to the invention, the introducer tool comprises a hollow body with a console or plate arranged at the introducer tool's entrance, said console or plate being devised with holes to hold surgical equipment, an optical instrument etc. in place.

According to another advantageous embodiment of the instrument according to the invention, the introducer tool is funnel-shaped, thereby facilitating its introduction. Here, the lateral channels are at an angle to the central channel.

According to an advantageous further developement of the instrument according to the invention, the end intended for introduction into the patient's body is supplied with a soft collar to prevent damage to the organ against which the tool is advanced.

According to yet another advantageous further developement of the instrument according to the invention, an adjustable spacer sleeve is provided on the introducer tool's exterior for setting the introducer tool's working distance. This ensures that the introducer tool is advanced a correct distance into each patient, a major advantage, since the correct distance for a particular operation can vary considerably from one patient to another.

According to other advantageous embodiments of the instrument according to the invention, the introducer tool has a round or oval cross-section.

Yet another advantageous embodiment of the invention, a mandrel, is introducible into the interior of the introducer tool to close off its leading end and expand the opening in the tissue to the heart or some other organ as the introducer tool is advanced into the patient's body, thereafter being removed from the introducer tool. This keeps blood and tissue from penetrating into the introducer tool during its advance into the patient's body.

One exemplification embodiment of the instrument according to the invention is described more in detail below, referring to the attached FIGS. 3 - 6.

The drawings illustrate the following:
FIG. 1 shows a surgical operation on a patient by means of conventional laparoscopy;
FIG. 2 shows a TV monitor with a picture of the surgical field in the heart;
FIG. 3 is a side view of one embodiment of the instrument according to the invention;
FIG. 4 is an end view of the instrument from the instrument's entrance end;
FIG. 5 shows an alternative embodiment of the instrument according to the invention, and
FIG. 6 shows an embodiment of a mandrel for the instrument according to the invention.

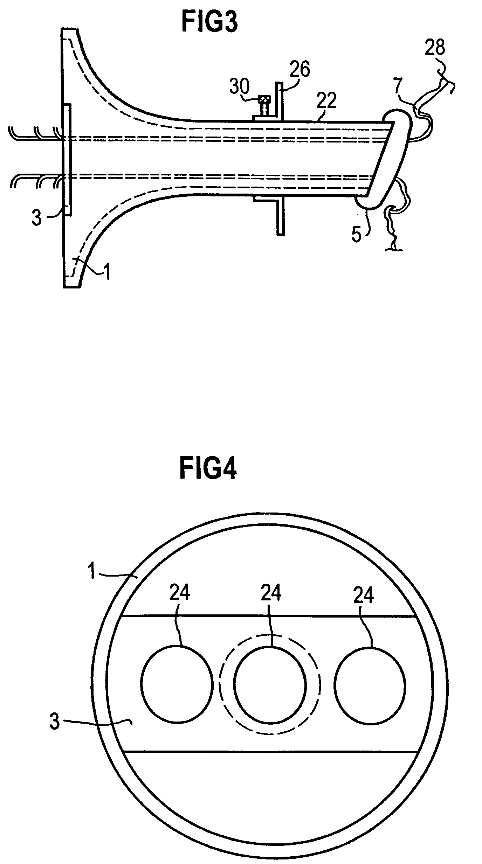

FIGS. 3 and 4 show a version of the instrument, according to the invention, with a funnel-shaped, single-tube introducer tool 1.

Over the introducer tool's 1 entrance is a holder in the form of a console 3 with holes 24 for different instruments and the like. Optical instruments for monitoring and inspecting the surgical field are suitably introduced through the central channel, whereas other instruments are introduced through lateral channels which are angled, in relation to the central channel, towards the narrow end of the funnel.

Instead of the illustrated console, a plate with an appropriate hole configuration can be arranged at the entrance end of the funnel. The hole configuration can obviously be varied as desired in an endless number of ways.

The funnel-shaped introducer tool 1 has a cylindrical section 2 on whose end a soft collar 5 is arranged to prevent the introducer tool from damaging the organ against which it is advanced during the laparoscopy.

With a spacer body means in the form of a sleeve 26, which is slidable on the introducer tool's exterior, the desired working distance can be set. The spacer means 26 can be locked with a locking screw 30 in the desired position on the introducer tool's 1 cylindrical part 22. This thereby ensures that the introducer tool 1 is advanced the correct distance into the patient, an important advantage, since the correct distance for any particular operation can vary considerably from one patient to another.

Hooks 7 can be introduced through the channels 24 in the introducer tool 1 for lifting and "hanging up" the pericardium 28 and even for securing the edge of the pericardium after the pericardium has been opened.

The external dimensions of the introducer tool can be about 40 mm, and the diameter of manipulation holes can typically be about 12 mm.

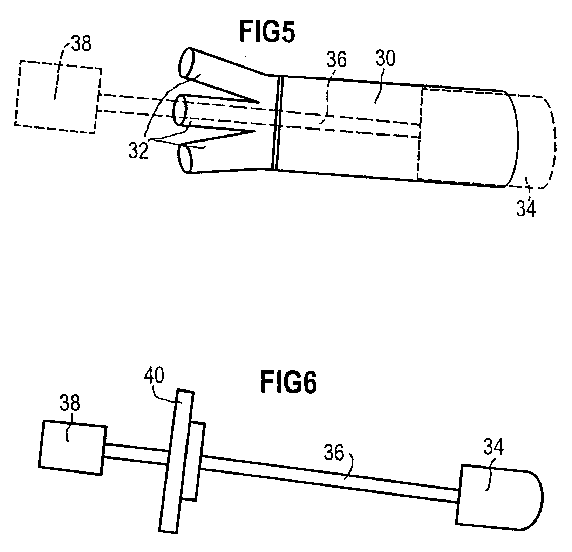

FIG. 5 shows an alternative version of an introducer tool, according to the invention, with a cylindrical section 30 intended for introduction into the patient's body. There are three guide fingers or guide cavities 32 arranged on the entrance side of the cylindrical section 30 through which surgical instruments, optical instruments and the like can be passed. The fingers 32 are then directed so the tools and instruments are oriented in the desired directions.

FIG. 6 shows a so-called mandrel designed for introduction into the introducer tool when the tool is to be introduced into the patient. The mandrel has a sealing body 34 whose shape is adapted to the shape of the introducer tool's interior leading end in order to close it off, preventing blood and body tissue from penetrating into the interior of the tool when it is advanced into the patient. The sealing body 34 is attached to a rod 36 with a handle 38 for introducing the sealing body 34 into the tool before the latter is introduced into the patient and for extracting the sealing body 34 after the introducer tool is in place. The rod 36 then passes through a hole in a plate 40 which is devised for placement over the entrance of the introducer tool to guide the 6 movements of the rod 36 inside the introducer tool. After the introducer tool is in place, the mandrel is removed completely.

The procedure for introducing stimulation electrodes into the heart with the instrument according to the invention can be as follows:
First, a subxiphoidal incision is made along the midline. The incision is widened and a channel opened through the diaphragm so the pericardium-clad cardiac apex becomes accessible.

The instrument according to the invention in then inserted into the channel and advanced until the soft collar is against the pericardium. Optical instruments are introduced through the central channel of the instruments, and a hook, whose progress is observed on a TV monitor, is introduced through e.g. a lateral channel, and the pericardium is hooked on to and lifted. Scissors are introduced through another channel in the instrument, and the pericardium is opened as much as necessary without affecting myocardium. One or more additional hooks are then introduced, and the edge of the pericardium is secured so the apex of the heart is exposed.

The optical instrument can then be inserted inside the pericardium and the entire surface of the heart can be inspected.

Electrodes are then introduced through introductory channels of the instrument and placed at the desired sites with appropriate tools under optical monitoring.

Finally, diagnostic equipment can be introduced through the instrument according to the invention for carrying out the required diagnostic measures.

It should be noted that the use of the instrument according to the invention is by no means limited to cardiac applications but can be employed for a number of other uses. The instrument according to the invention can be utilized subcutaneously and e.g. intraabdominally.

## Claims

1. An instrument for laparoscopy, characterized in that an introducer tool (1) is devised for introduction and positional fixation of surgical equipment in the form of at least two surgical tools, optical instruments, implants or the like.

2. An instrument of claim 1, characterized in that the introducer tool (1) is devised with a channel for the introduction of an optical instrument and additional channels for the introduction of other surgical equipment.

3. An instrument of claim 1, characterized in that the introducer tool comprises a hollow body with a console (3) or plate arranged at the entrance end of the introducer tool (1), said console or plate being devised with holes to hold surgical equipment and optical instruments.

4. An instrument of any of claims 1 to 5, characteized in that the introducer tool (1) is funnel-shaped.

5. An instrument of claims 2 and 4, characterized in that the lateral channels are at an angle to the central channel.

6. An instrument of claim 1, characterized in that the introducer tool comprises a hollow body at whose entrance a plurality of hollow, appropriately directed guide cavities are arranged through which surgical tools, optical instruments and the like can be introduced and guided in the desired introduction direction.

7. An instrument of any of claims 1 - 6, characterized in that the end of the introducer tool (1) intended for introduction into the body has a soft bearing collar (5).

8. An instrument of any of claims 1 - 7, characterized in that an adjustable spacer sleeve (26) adjustable on the exterior of the introducer tool (1) is arranged to determine the working distance of the introducer tool.

9. An instrument of any of claims 1 - 8, characterized in that the introducer tool (1) has a round cross-section.

10. An instrument of any of claims 1 - 8, characterized in that the introducer tool (1) has an oval cross-section.

11. An instrument of any of claims 1, 3 or 7 - 10, characterized in that a mandrel is introducible into the interior of the introducer tool to close off its leading end and expand the opening in the tissue to the heart or some other organ as the introducer tool is advanced into the patient's body, and thereafter is removed from the introducer tool.

12. An instrument of claim 11, characterized in that the mandrel comprises a sealing body, attached to a rod for inserting the mandrel into and removing the mandrel from the introducer tool, said sealing body being adapted to the leading end section of the introducer tool.
